# EUROPEAN PATENT SPECIFICATION

(11) **EP 3 323 888 B1**
(45) Date of publication and mention of the grant of the patent: **21.08.2019**
(21) Application number: 17201764.2
(22) Date of filing: 15.11.2017
(51) Int. Cl.: C12N 9/12, C12P 19/04

(54) **MICROORGANISM OF THE GENUS KOMAGATAEIBACTER HAVING ENHANCED CELLULOSE PRODUCTIVITY, METHOD OF PRODUCING CELLULOSE USING THE SAME, AND METHOD OF PRODUCING THE MICROORGANISM**
MIKROORGANISMUS DER GATTUNG KOMAGATAEIBACTER MIT VERBESSERTER CELLULOSEPRODUKTIVITÄT, VERFAHREN ZUR HERSTELLUNG VON CELLULOSE DAMIT UND VERFAHREN ZUR HERSTELLUNG DES MIKROORGANISMUS
MICRO-ORGANISME DU GENRE KOMAGATAEIBACTER PRÉSENTANT UNE MEILLEURE PRODUCTIVITÉ DE CELLULOSE, PROCÉDÉ DE PRODUCTION DE CELLULOSE UTILISANT CELUI-CI, ET PROCÉDÉ DE PRODUCTION DU MICRO-ORGANISME

(30) Priority: 21.11.2016 KR 20160154878
(43) Date of publication of application: 23.05.2018
(73) Proprietor: Samsung Electronics Co., Ltd., Gyeonggi-do 16677 (KR)
(72) Inventor: Chung, Soonchun, Gyeonggi-do 16678 (KR); Yun, Jiae, Gyeonggi-do 16678 (KR); Kang, Jinkyu, Gyeonggi-do 16678 (KR); Park, Jinhwan, Gyeonggi-do 16678 (KR)
(74) Representative: Grünecker Patent- und Rechtsanwälte PartG mbB

(56) References cited:
- US-A1- 2006 040 365
- NAKAI TOMONORI ET AL: "Enhancement of cellulose production by expression of sucrose synthase in Acetobacter xylinum", PROCEEDINGS OF THE NATIONAL ACADEMY OF SCIENCES OF THE UNITED STATES OF AMERICA,, vol. 96, no. 1, 5 January 1999 (1999-01-05), pages 14-18, XP002557653, DOI: 10.1073/PNAS.96.1.14
- TONOUCHI NAOTO ET AL: "Characterization of the biosynthetic pathway of cellulose from glucose and fructose in Acetobacter xylinum", BIOSCIENCE BIOTECHNOLOGY AND BIOCHEMISTRY, vol. 60, no. 8, 1996, pages 1377-1379, XP002778988, ISSN: 0916-8451

<110> Samsung Electronics Co., Ltd.
<120> Komagataeibacter genus microorganism having enhanced cellulose productivity, method for producing cellulose using the same, and method for producing the microorganism
<130> EP114505FZSEpau
<140> not yet assigned
   <141> herewith
<150> KR10-2016-0154878
   <151> 2016-11-21
<160> 19
<170> KopatentIn 2.0
<210> 1
   <211> 320
   <212> PRT
   <213> E.coli
<400> 1
   Met Ile Lys Lys Ile Gly Val Leu Thr Ser Gly Gly Asp Ala Pro Gly
    1               5                  10                  15
   Met Asn Ala Ala Ile Arg Gly Val Val Arg Ser Ala Leu Thr Glu Gly
               20                  25                  30
   Leu Glu Val Met Gly Ile Tyr Asp Gly Tyr Leu Gly Leu Tyr Glu Asp
           35                  40                  45
   Arg Met Val Gln Leu Asp Arg Tyr Ser Val Ser Asp Met Ile Asn Arg
       50                  55                  60
   Gly Gly Thr Phe Leu Gly Ser Ala Arg Phe Pro Glu Phe Arg Asp Glu
   65                  70                  75                  80
   Asn Ile Arg Ala Val Ala Ile Glu Asn Leu Lys Lys Arg Gly Ile Asp
                   85                  90                  95
   Ala Leu Val Val Ile Gly Gly Asp Gly Ser Tyr Met Gly Ala Met Arg
               100                 105                 110
   Leu Thr Glu Met Gly Phe Pro Cys Ile Gly Leu Pro Gly Thr Ile Asp
           115                 120                 125
   Asn Asp Ile Lys Gly Thr Asp Tyr Thr Ile Gly Phe Phe Thr Ala Leu
       130                 135                 140
   Ser Thr Val Val Glu Ala Ile Asp Arg Leu Arg Asp Thr Ser Ser Ser
   145                 150                 155                 160
   His Gln Arg Ile Ser Val Val Glu Val Met Gly Arg Tyr Cys Gly Asp
                   165                 170                 175
   Leu Thr Leu Ala Ala Ala Ile Ala Gly Gly Cys Glu Phe Val Val Val
               180                 185                 190
   Pro Glu Val Glu Phe Ser Arg Glu Asp Leu Val Asn Glu Ile Lys Ala
           195                 200                 205
   Gly Ile Ala Lys Gly Lys Lys His Ala Ile Val Ala Ile Thr Glu His
       210                 215                 220
   Met Cys Asp Val Asp Glu Leu Ala His Phe Ile Glu Lys Glu Thr Gly
   225                 230                 235                 240
   Arg Glu Thr Arg Ala Thr Val Leu Gly His Ile Gln Arg Gly Gly Ser
                   245                 250                 255
   Pro Val Pro Tyr Asp Arg Ile Leu Ala Ser Arg Met Gly Ala Tyr Ala
               260                 265                 270
   Ile Asp Leu Leu Leu Ala Gly Tyr Gly Gly Arg Cys Val Gly Ile Gln
           275                 280                 285
   Asn Glu Gln Leu Val His His Asp Ile Ile Asp Ala Ile Glu Asn Met
       290                 295                 300
   Lys Arg Pro Phe Lys Gly Asp Trp Leu Asp Cys Ala Lys Lys Leu Tyr
   305                 310                 315                 320
<210> 2
   <211> 963
   <212> DNA
   <213> E.coli
<400> 2
   atgattaaga aaatcggtgt gttgacaagc ggcggtgatg cgccaggcat gaacgccgca      60
   attcgcgggg ttgttcgttc tgcgctgaca gaaggtctgg aagtaatggg tatttatgac     120
   ggctatctgg gtctgtatga agaccgtatg gtacagctag accgttacag cgtgtctgac     180
   atgatcaacc gtggcggtac gttcctcggt tctgcgcgtt tcccggaatt ccgcgacgag     240
   aacatccgcg ccgtggctat cgaaaacctg aaaaaacgtg gtatcgacgc gctggtggtt     300
   atcggcggtg acggttccta catgggtgca atgcgtctga ccgaaatggg cttcccgtgc     360
   atcggtctgc cgggcactat cgacaacgac atcaaaggca ctgactacac tatcggtttc     420
   ttcactgcgc tgagcaccgt tgtagaagcg atcgaccgtc tgcgtgacac ctcttcttct     480
   caccagcgta tttccgtggt ggaagtgatg ggccgttatt gtggagatct gacgttggct     540
   gcggccattg ccggtggctg tgaattcgtt gtggttccgg aagttgaatt cagccgtgaa     600
   gacctggtaa acgaaatcaa agcgggtatc gcgaaaggta aaaaacacgc gatcgtggcg     660
   attaccgaac atatgtgtga tgttgacgaa ctggcgcatt tcatcgagaa agaaaccggt     720
   cgtgaaaccc gcgcaactgt gctgggccac atccagcgcg gtggttctcc ggtgccttac     780
   gaccgtattc tggcttcccg tatgggcgct tacgctatcg atctgctgct ggcaggttac     840
   ggcggtcgtt gtgtaggtat ccagaacgaa cagctggttc accacgacat catcgacgct     900
   atcgaaaaca tgaagcgtcc gttcaaaggt gactggctgg actgcgcgaa aaaactgtat     960
   taa                                                                   963
<210> 3
   <211> 39
   <212> DNA
   <213> Artificial Sequence
<220>
   <223> PFKA primer
<400> 3
   cgtacccggg gatccatgat taagaaaatc ggtgtgttg 39
<210> 4
   <211> 39
   <212> DNA
   <213> Artificial Sequence
<220>
   <223> PFKA primer
<400> 4
   gactctagag gatccttaat acagtttttt cgcgcagtc 39
<210> 5
   <211> 33
   <212> DNA
   <213> Artificial Sequence
<220>
   <223> F1 forward primer
<400> 5
   cggcgtagag gatcaggagc ttatcgactg cac 33
<210> 6
   <211> 28
   <212> DNA
   <213> Artificial Sequence
<220>
   <223> F1 reverse primer
<400> 6
   ccggcgtaga gaatccacag gacgggtg 28
<210> 7
   <211> 27
   <212> DNA
   <213> Artificial Sequence
<220>
   <223> F2 forward primer
<400> 7
   ctgtggattc tctacgccgg acgcatc 27
<210> 8
   <211> 29
   <212> DNA
   <213> Artificial Sequence
<220>
   <223> F2 reverse primer
<400> 8
   aagggcatcg gtcgtcgctc tcccttatg 29
<210> 9
   <211> 3576
   <212> DNA
   <213> Artificial Sequence
<220>
   <223> pTSa-EX1 vector
<400> 9
   gaattcagcc agcaagacag cgatagaggg tagttatcca cgtgaaaccg ctaatgcccc 60
   gcaaagcctt gattcacggg gctttccggc ccgctccaaa aactatccac gtgaaatcgc 120
   taatcagggt acgtgaaatc gctaatcgga gtacgtgaaa tcgctaataa ggtcacgtga 180
   aatcgctaat caaaaaggca cgtgagaacg ctaatagccc tttcagatca acagcttgca 240
   aacacccctc gctccggcaa gtagttacag caagtagtat gttcaattag cttttcaatt 300
   atgaatatat atatcaatta ttggtcgccc ttggcttgtg gacaatgcgc tacgcgcacc 360
   ggctccgccc gtggacaacc gcaagcggtt gcccaccgtc gagcgccagc gcctttgccc 420
   acaacccggc ggccggccgc aacagatcgt tttataaatt tttttttttg aaaaagaaaa 480
   agcccgaaag gcggcaacct ctcgggcttc tggatttccg atcacctgta agtcggacgt 540
   tccgatcacc tgtaacgatg cgtccggcgt agaggatccg gagcttatcg actgcacggt 600
   gcaccaatgc ttctggcgtc aggcagccat cggaagctgt ggtatggctg tgcaggtcgt 660
   aaatcactgc ataattcgtg tcgctcaagg cgcactcccg ttctggataa tgttttttgc 720
   gccgacatca taacggttct ggcaaatatt ctgaaatgag ctgttgacaa ttaatcatcg 780
   gctcgtataa tgtgtggaat tgtgagcgga taacaatttc acacagggac gagctattga 840
   ttgggtaccg agctcgaatt cgtacccggg gatcctctag agtcgacctg caggcatgca 900
   agcttggctg ttttggcgga tgagagaaga ttttcagcct gatacagatt aaatcagaac 960
   gcagaagcgg tctgataaaa cagaatttgc ctggcggcag tagcgcggtg gtcccacctg 1020
   accccatgcc gaactcagaa gtgaaacgcc gtagcgccga tggtagtgtg gggtctcccc 1080
   atgcgagagt agggaactgc caggcatcaa ataaaacgaa aggctcagtc gaaagactgg 1140
   gcctttcgtt ttatctgttg tttgtcggtg aacgctctcc tgagtaggac aaatccgccg 1200
   ggagcggatt tgaacgttgc gaagcaacgg cccggagggt ggcgggcagg acgcccgcca 1260
   taaactgcca ggcatcaaat taagcagaag gccatcctga cggatggcct ttttgcaaga 1320
   acatgtgagc acttccgctt cctcgctcac tgactcgctg cgctcggtcg ttcggctgcg 1380
   gcgagcggta tcagctcact caaaggcggt aatacggtta tccacagaat caggggataa 1440
   cgcaggaaag aacatgtgag caaaaggcca gcaaaaggcc aggaaccgta aaaaggccgc 1500
   gttgctggcg tttttccata ggctccgccc ccctgacgag catcacaaaa atcgacgctc 1560
   aagtcagagg tggcgaaacc cgacaggact ataaagatac caggcgtttc cccctggaag 1620
   ctccctcgtg cgctctcctg ttccgaccct gccgcttacc ggatacctgt ccgcctttct 1680
   cccttcggga agcgtggcgc tttctcatag ctcacgctgt aggtatctca gttcggtgta 1740
   ggtcgttcgc tccaagctgg gctgtgtgca cgaacccccc gttcagcccg accgctgcgc 1800
   cttatccggt aactatcgtc ttgagtccaa cccggtaaga cacgacttat cgccactggc 1860
   agcagccact ggtaacagga ttagcagagc gaggtatgta ggcggtgcta cagagttctt 1920
   gaagtggtgg cctaactacg gctacactag aagaacagca tttggtatct gcgctctgct 1980
   gaagccagtt accttcggaa aaagagttgg tagctcttga tccggcaaac aaaccaccgc 2040
   tggtagcggt ggtttttttg tttgcaagca gcagattacg cgcagaaaaa aaggatctca 2100
   agaagatcct ttgatctttt ctacggggtc tgacgctcag tggaacgaaa actcacgtta 2160
   attctcatgt ttgacagctt atcatcgata agctttaatg cggtagttta tcacagttaa 2220
   attgctaacg cagtcaggca ccgtgtatga aatctaacaa tgcgctcatc gtcatcctcg 2280
   gcaccgtcac cctggatgct gtaggcatag gcttggttat gccggtactg ccgggcctct 2340
   tgcgggatat cgtccattcc gacagcatcg ccagtcacta tggcgtgctg ctagcgctat 2400
   atgcgttgat gcaatttcta tgcgcacccg ttctcggagc actgtccgac cgctttggcc 2460
   gccgcccagt cctgctcgct tcgctacttg gagccactat cgactacgcg atcatggcga 2520
   ccacacccgt cctgtggatc ctctacgccg gacgcatcgt ggccggcatc accggcgcca 2580
   caggtgcggt tgctggcgcc tatatcgccg acatcaccga tggggaagat cgggctcgcc 2640
   acttcgggct catgagcgct tgtttcggcg tgggtatggt ggcaggcccc gtggccgggg 2700
   gactgttggg cgccatctcc ttgcatgcac cattccttgc ggcggcggtg ctcaacggcc 2760
   tcaacctact actgggctgc ttcctaatgc aggagtcgca taagggagag cgtcgaccga 2820
   tgcccttgag agccttcaac ccagtcagct ccttccggtg ggcgcggggc atgactatcg 2880
   tcgccgcact tatgactgtc ttctttatca tgcaactcgt aggacaggtg ccggcagcgc 2940
   tctgggtcat tttcggcgag gaccgctttc gctggagcgc gacgatgatc ggcctgtcgc 3000
   ttgcggtatt cggaatcttg cacgccctcg ctcaagcctt cgtcactggt cccgccacca 3060
   aacgtttcgg cgagaagcag gccattatcg ccggcatggc ggccgacgcg ctgggctacg 3120
   tcttgctggc gttcgcgacg cgaggctgga tggccttccc cattatgatt cttctcgctt 3180
   ccggcggcat cgggatgccc gcgttgcagg ccatgctgtc caggcaggta gatgacgacc 3240
   atcagggaca gcttcaagga tcgctcgcgg ctcttaccag cctaacttcg atcactggac 3300
   cgctgatcgt cacggcgatt tatgccgcct cggcgagcac atggaacggg ttggcatgga 3360
   ttgtaggcgc cgccctatac cttgtctgcc tccccgcgtt gcgtcgcggt gcatggagcc 3420
   gggccacctc gacctgaatg gaagccggcg gcacctcgct aacggattca ccactccaag 3480
   aattggagcc aatttttaag gcagttattg gtgcccttaa acgcctggtt gctacgcctg 3540
   aataagtgat aataagcgga tgaatggcag aaattc 3576
<210> 10
   <211> 40
   <212> DNA
   <213> Artificial Sequence
<220>
   <223> primer
<400> 10
   cttgatatcg aattcttctc atgtttgaca gcttatcatc 40
<210> 11
   <211> 36
   <212> DNA
   <213> Artificial Sequence
<220>
   <223> primer
<400> 11
   gggctgcagg aattcgaatt tctgccattc atccgc 36
<210> 12
   <211> 37
   <212> DNA
   <213> Artificial Sequence
<220>
   <223> primer
<400> 12
   cttgatatcg aattaggcct gtcatcgtct atatacg 37
<210> 13
   <211> 42
   <212> DNA
   <213> Artificial Sequence
<220>
   <223> primer
<400> 13
   cgtgttgttc gaattcgatg gatattcctc cagtatcatg tg 42
<210> 14
   <211> 32
   <212> DNA
   <213> Artificial Sequence
<220>
   <223> primer
<400> 14
   catcgaattc gaacaacacg ccgatgtatg ac 32
<210> 15
   <211> 40
   <212> DNA
   <213> Artificial Sequence
<220>
   <223> primer
<400> 15
   acatgagaag aattgacaga tccggtcagt tcacattatc 40
<210> 16
   <211> 39
   <212> DNA
   <213> Artificial Sequence
<220>
   <223> primer
<400> 16
   cagaaattcg aattgcgatc atcaccaacc aggaaattc 39
<210> 17
   <211> 37
   <212> DNA
   <213> Artificial Sequence
<220>
   <223> primer
<400> 17
   gggctgcagg aattgggtat ttcaggcggc agtaaag 37
<210> 18
   <211> 40
   <212> DNA
   <213> Artificial Sequence
<220>
   <223> primer
<400> 18
   cttgatatcg aattcttctc atgtttgaca gcttatcatc 40
<210> 19
   <211> 36
   <212> DNA
   <213> Artificial Sequence
<220>
   <223> primer
<400> 19
   gggctgcagg aattcgaatt tctgccattc atccgc 36

## Description

### BACKGROUND

### 1. Field

The present disclosure relates to a microorganism of the genus *Komagataeibacter* having enhanced cellulose productivity, a method of producing cellulose by using the same, and a method of producing the microorganism.

### 2. Description of the Related Art

Cellulose produced by culturing microorganisms exists as a primary structure of β-1,4 glucan composed of glucose, and β-1,4-glucans form a network structure of fibril bundles. This cellulose is also called 'biocellulose' or 'microbial cellulose'.

This microbial cellulose is pure cellulose entirely free of lignin or hemicellulose, unlike plant cellulose. The microbial cellulose is 100 nm or less in width, and has characteristics of high water absorption and retention capacity, high tensile strength, high elasticity, high heat resistance, etc, compared to plant cellulose. Due to these characteristics, microbial cellulose has been developed by application to a variety of fields, such as cosmetics, medical products, dietary fibers, audio speaker diaphragms, functional films, etc.

As a microbial cellulose-producing strain, *Acetobacter, Agrobacteria, Rhizobia,* or *Sarcina* has been reported. Of them, *Komagataeibacter xylinum* (also called *'Gluconacetobacter xylinum*') is known as an excellent strain. Upon static culture under aerobic conditions, cellulose with a three-dimensional network structure is formed as a thin film on the surface of a culture medium. Nakai Tomonori et al. (1999, PNAS 96, 14-18) discloses enhanced cellulose production in Acetobacter xylinum by genetic transformation with E. coli sucrose synthase.

Phosphofructose kinase (PFK) is a protein that phosphorylates fructose-6-phosphate into fructose-1,6-bisphosphate (FBP) in the corresponding reaction. PFK plays a key regulatory role in the corresponding reaction. PFK is allosterically inhibited by ATP and allosterically activated by AMP, thus indicating the cell's energetic needs when it undergoes the corresponding reaction. PFK exists as a homotetramer in bacteria and mammals and as an octomer in yeast.

Although studies for improving cellulose productivity by inducing various mutations in a cellulose production strain have been conducted, there is a demand for a method of producing a microorganism of the genus *Komagataeibacter* having enhanced cellulose productivity, despite the above-described prior art.

### SUMMARY

An aspect provides a microorganism of the genus *Komagataeibacter* having enhanced cellulose productivity.

Another aspect provides a method of producing cellulose by using the microorganism.

Still another aspect provides a method of producing the microorganism.

### BRIEF DESCRIPTION OF THE DRAWINGS

These and/or other aspects will become apparent and more readily appreciated from the following description of the embodiments, taken in conjunction with the accompanying drawings in which:
FIG. 1 shows cellulose nanofiber (CNF) production of *K.xylinus* strain into which a pfkA gene is introduced;
FIG. 2 shows CNF yield of *K.xylinus* strain into which a pfkA gene is introduced;
FIG. 3 shows CNF production and yield of *K.xylinus* strain into which a pfkA gene is introduced during fermentation in a medium free of carboxy methyl cellulose (CMC); and
FIG. 4 shows CNF production and yield of *K.xylinus* strain into which a pfkA gene is introduced during fermentation in a medium including CMC.

### DETAILED DESCRIPTION

The term "increase in activity" or "increased activity", as used herein, may refer to a detectable increase in an activity of a cell, a protein, or an enzyme. The "increase in activity" or "increased activity" may also refer to an activity level of a modified (e.g., genetically engineered) cell, protein, or enzyme that is higher than that of a comparative cell, protein, or enzyme of the same type, such as a cell, protein, or enzyme that does not have a given genetic modification (e.g., original or "wild-type" cell, protein, or enzyme). "Cell activity" may refer to an activity of a particular protein or enzyme of a cell. For example, an activity of a modified or engineered cell may be increased by about 5% or more, about 10% or more, about 15% or more, about 20% or more, about 30% or more, about 50% or more, about 60% or more, about 70% or more, or about 100% or more than an activity of a non-engineered cell or a parent cell of the same type, i.e., a particular protein or enzyme of a wild-type cell. A cell having an increased activity of a protein or an enzyme may be identified by using any method known in the art.

An increase in an activity of an enzyme or a polypeptide may be achieved by an increase in expression or specific activity. The increase in expression may be caused by introduction of a polynucleotide encoding the enzyme or the polypeptide into a cell, by increase of the copy number thereof, or by modification of a regulatory region of the polypeptide. A microorganism into which the polynucleotide encoding the enzyme is introduced may be a microorganism endogenously including the polynucleotide or not. The polynucleotide encoding the enzyme may be operably linked to a regulatory sequence that allows expression thereof, for example, a promoter, a polyadenylation site, or a combination thereof. The polynucleotide whose copy number is increased may be endogenous or exogenous. The endogenous gene refers to a gene which has existed on a genetic material included in a microorganism. The exogenous gene refers to a gene that is externally introduced into a cell, and may be homologous or heterologous with respect to a host cell into which the gene is introduced. The term "heterologous" means "foreign" or "not native" to the species.

The term "increase of the copy number" may be caused by introduction or amplification of the gene, and may be achieved by genetically engineering a cell so that the cell is allowed to have a gene that does not exist in a non-engineered cell. The introduction of the gene may be mediated by a vehicle such as a vector. The introduction may be a transient introduction in which the gene is not integrated into a genome, or an introduction that results in integration of the gene into the genome. The introduction may be performed, for example, by introducing a vector into the cell, the vector including a polynucleotide encoding a target polypeptide, and then, replicating the vector in the cell, or by integrating the polynucleotide into the genome.

The introduction of the gene may be performed via a known method, for example, transformation, transfection, or electroporation. The gene may be introduced via a vehicle or as it is. The term "vehicle" or "vector", as used herein, refers to a nucleic acid molecule that is able to deliver other nucleic acids linked thereto into a cell. As a nucleic acid sequence mediating introduction of a specific gene, the vehicle used may be a vector, a nucleic acid construct, or a cassette. The vector may include, for example, a plasmid vector, a virus-derived vector, etc. The vector may include, for example, a plasmid expression vector, a virus expression vector, such as a replication-defective retrovirus, adenovirus, adeno-associated virus, or a combination thereof.

The genetic modification used in the present disclosure may be performed by a molecular biological method known in the art.

The term "parent cell" refers to an original cell, for example, a non-genetically engineered cell of the same type as an engineered microorganism. With respect to a particular genetic modification, the "parent cell" may be a cell that lacks the particular genetic modification, but is identical in all other respects. Thus, the parent cell may be a cell that is used as a starting material to produce a genetically engineered microorganism having an increased activity of a given protein (e.g., a protein having a sequence identity of about 90% or higher with respect to phosphofructose kinase (PFK)). In addition, with respect to a microorganism having an enhanced activity of PFK in a cell due to genetic modification of a gene encoding PFK, the parent cell may be a microorganism that is not genetically modified. The same comparison is also applied to other genetic modifications.

The term "gene", as used herein, refers to a nucleic acid fragment encoding a particular protein, and may include or not a regulatory sequence of a 5'-non coding sequence and/or a 3'-non coding sequence.

The term "sequence identity" of a polynucleotide or a polypeptide, as used herein, refers to a degree of identity between bases or amino acid residues of sequences obtained after the sequences are aligned so as to best match in certain comparable regions. The sequence identity is a value that is measured by comparing two sequences in certain comparable regions via optimal alignment of the two sequences, in which portions of the sequences in the certain comparable regions may be added or deleted compared to reference sequences. A percentage of sequence identity may be calculated by, for example, comparing two optimally aligned sequences in the entire comparable regions, determining the number of locations in which the same amino acids or nucleic acids appear to obtain the number of matching locations, dividing the number of matching locations by the total number of locations in the comparable regions (that is, the size of a range), and multiplying a result of the division by 100 to obtain the percentage of the sequence identity. The percentage of the sequence identity may be determined using a known sequence comparison program, for example, BLASTN (NCBI), BLASTP (NCBI), CLC Main Workbench (CLC bio), MegAlign™ (DNASTAR Inc), etc.

Various levels of sequence identity may be used to identify various types of polypeptides or polynucleotides having the same or similar functions or activities. For example, the sequence identity may include a sequence identity of about 50% or more, about 55% or more, about 60% or more, about 65% or more, about 70% or more, about 75% or more, about 80% or more, about 85% or more, about 90% or more, about 95% or more, about 96% or more, about 97% or more, about 98% or more, about 99% or more, or 100%.

The term "genetic modification", as used herein, refers to an artificial alteration in a constitution or structure of a genetic material of a cell.

An aspect of the disclosure provides a microorganism of the genus *Komagataeibacter* having enhanced cellulose productivity and including a genetic modification that increases a phosphofructose kinase (PFK) activity.

PFK is a protein that phosphorylates fructose-6-phosphate into fructose-1,6-bisphosphate in the corresponding reaction. PFK may be exogenous or endogenous. PFK may be PFK1 (also, referred to as "PFKA"). PFK may belong to the enzyme classified as EC 2.7.1.11. PFK may be derived from bacteria. PFK may be derived from the genus *Escherichia,* genus *Bacillus,* genus *Mycobacterium,* genus *Zymomonas,* or genus *Vibrio.* PFK may be derived from *E. coli,* for example, *E.coli* MG1655.

PFK may catalyze conversion of ATP and fructose-6-phosphate into fructose-1,6-bisphosphate and ADP. PFK may be allosterically activated by ADP and diphosphonucleoside and allosterically inhibited by phosphoenolpyruvate. PFK may be a polypeptide having a sequence identity of about 90% or higher, about 95% or higher, or about 100% with an amino acid sequence of SEQ ID NO: 1

In the microorganism, the genetic modification may increase expression of a gene encoding PFK. The genetic modification may be an increase of the copy number of a gene encoding the PFK or a modification of an expression regulatory sequence of a gene encoding the PFK. The increase of the copy number may be caused by introduction of the gene into a cell from the outside or by amplification of an endogenous gene. The gene may be a polynucleotide encoding PFK that belongs to the enzyme classified as EC 2.7.1.11. The PFK may be derived from bacteria. The gene may be derived from the genus *Escherichia,* genus *Bacillus,* genus *Mycobacterium,* genus *Zymomonas,* or genus *Vibrio.* The gene may be derived from *E. coli.* The gene may have a nucleotide sequence encoding an amino acid sequence having a sequence identity of about 90%, 95%, or 99% with an amino acid sequence of SEQ ID NO: 1. The gene may have a sequence identity of about 90% or more, about 95% or more, or about 100% or more of a nucleotide sequence of SEQ ID NO: 2.

The genetic modification may introduce the gene encoding the PFK, for example, via a vehicle such as a vector. The gene encoding the PFK may exist within or outside the chromosome. The introduced gene encoding the PFK may be a plurality of genes, for example, 2 or more, 5 or more, 10 or more, 30 or more, 50 or more, 100 or more, or 1000 or more.

The microorganism may belong to the genus *Komagataeibacter* and may have bacterial cellulose productivity. The microorganism may not have endogenous PFK. Thus, the microorganism may not have an endogenous corresponding reaction, which is a glycolytic pathway. The microorganism may be *K. xylinus* (also, referred to as "*G. xylinus*"), *K.rhaeticus, K. swingsii, K. kombuchae, K.nataicola,* or *K. sucrofermentans.*

Another aspect of the disclosure provides a method of producing cellulose, the method including culturing a microorganism of the genus *Komagataeibacter* having enhanced cellulose productivity and comprising a genetic modification that increases a PFK activity in a medium to produce cellulose; and collecting the cellulose from a culture.

The method includes the culturing of a microorganism of the genus *Komagataeibacter* having enhanced cellulose productivity and comprising a genetic modification that increases a PFK activity in a medium to produce cellulose. The microorganism of the genus *Komagataeibacter* may be produced as follows.

The culturing may be performed in a medium containing a carbon source, for example, glucose. The medium used for culturing the microorganism may be any general medium suitable for host cell growth, such as a minimal or complex medium containing appropriate supplements. The suitable medium may be commercially available or prepared by a known preparation method.

The medium may be a medium that may satisfy the requirements of a particular microorganism depending on a selected product of culturing. The medium may be a medium including components selected from the group consisting of a carbon source, a nitrogen source, a salt, trace elements, and combinations thereof.

The culturing conditions may be appropriately controlled for the production of a selected product, for example, cellulose. The culturing may be performed under aerobic conditions for cell proliferation. The culturing may be performed by spinner culture or static culture without shaking. A density of the microorganism may be a density which gives enough space so as not to disturb production of cellulose.

The term "culture conditions", as used herein, mean conditions for culturing the microorganism. Such culture conditions may include, for example, a carbon source, a nitrogen source, or an oxygen condition utilized by the microorganism. The carbon source that may be utilized by the microorganism may include monosaccharides, disaccharides, or polysaccharides. The carbon source may include glucose, fructose, mannose, or galactose as an assimilable glucose. The nitrogen source may be an organic nitrogen compound or an inorganic nitrogen compound. The nitrogen source may be exemplified by amino acids, amides, amines, nitrates, or ammonium salts. An oxygen condition for culturing the microorganism may be an aerobic condition of a normal oxygen partial pressure or a low-oxygen condition including about 0.1% to about 10% of oxygen in the atmosphere. A metabolic pathway may be modified in accordance with a carbon source or a nitrogen source that may be actually used by a microorganism.

The medium may include ethanol or cellulose. The ethanol may be about 0.1 to 5%(v/v), for example, about 0.3 to 2.5%(v/v), about 0.3 to 2.0%(v/v), about 0.3 to 1.5%(v/v), about 0.3 to 1.25%(v/v), about 0.3 to 1.0%(v/v), about 0.3 to 0.7%(v/v), or about 0.5 to 3.0%(v/v) with respect to a volume of the medium. The cellulose may be about 0.5 to 5%(w/v), about 0.5 to 2.5%(w/v), about 0.5 to 1.5%(w/v), or about 0.7 to 1.25%(w/v) with respect to a volume of the medium. The cellulose may be carboxylated cellulose. The cellulose may be CMC. The CMC may be sodium CMC.

The method may include separating the cellulose from the culture. The separating may be, for example, collecting of a cellulose pellicle formed on the top of the medium. The cellulose pellicle may be collected by physically stripping off the cellulose pellicle or by removing the medium. The separating may be collecting of the cellulose pellicle while maintaining its shape without damage.

Another aspect of the disclosure provides a method of producing a microorganism having enhanced cellulose productivity, the method including introducing a gene encoding a PKF into a microorganism of the genus *Komagataeibacter.* The introducing of a gene encoding a PKF may be introducing a vehicle including the gene into the microorganism. In the method, the genetic modification may include amplifying the gene, engineering a regulatory sequence of the gene, or engineering a sequence of the gene itself. The engineering may be insertion, substitution, conversion, or addition of a nucleotide.

According to an embodiment, the recombinant microorganism of the genus *Komagataeibacter* having enhanced cellulose productivity may be used to produce cellulose at a high efficiency.

According to another embodiment, a method of producing cellulose may be used to efficiently produce cellulose.

According to another embodiment, a method of producing a microorganism having enhanced cellulose productivity may be used to efficiently produce a microorganism having enhanced cellulose productivity.

Hereinafter, the present invention will be described in more detail with reference to Examples. However, these Examples are provided for illustrative purposes only, and the invention is not intended to be limited by these Examples.

### Example 1. Preparation of K.xylinus including phosphofructose kinase (PFK) gene and Production of Cellulose

In this Example, *Komagataeibacter xylinus* DSM2325(DSM, Germany) was introduced with an exogenous PFK gene, and the microorganism introduced with the gene was cultured so that the microorganism consumed glucose and produced cellulose, thereby examining effects of the gene introduction on cellulose productivity.

### 1. Preparation of vector for over-expressing pfkA

The phosphofructose kinase (pfk) gene in *K.xylinus* was introduced by homologous recombination. A specific procedure is as follows.

An amplification product was obtained by PCR amplification using a pTSa-EX1 vector (SEQ ID NO: 9) as a template and a set of primers of SEQ ID NO: 5 and SEQ ID NO: 6 and a set of primers of SEQ ID NO: 7 and SEQ ID NO: 8. The amplification product was cloned by using an In-Fusion GD cloning kit (Takara) at the BamHI and SalI restriction sites of the pTSa-EX1 vector. The pTSa-EX1 vector is a shuttle vector which replicates in both *E. coli* and *X. xylinus.*

In order to introduce pfkA by homologous recombination, an open reading frame (ORF) (SEQ ID NO: 2) of the pfkA gene was produced by PCR amplification using a genome DNA of *E. coli* K12 MG1655 as a template and a set of primers of SEQ ID NO: 3 and SEQ ID NO: 4 as primers. Fragments of the pfkA gene were cloned at the BamHI and SalI restriction enzyme sites of the pTSa-EX11 vector by using an In-Fusion GD cloning kit (Takara) to prepare vector pTSa-Ec.pfkA for over-expressing pfkA.

### 2. Preparation of vector for inserting E. coli pfkA gene

A tetA gene was amplified by PCR amplification using a pTSa-Ec.pfkA vector as a template and a set of primers of SEQ ID NO: 10 and SEQ ID NO: 11 as primers. The PCR product was cloned at a EcoRI restriction enzyme site of a pMSK+ vector (Genbank Accession No. KJ922019) by using an In-fusion GD cloning kit (Takara) to prepare a pTSK+ vector.

A homologous region of a site to which a pfkA gene was about to be inserted was amplified by PCR using a genome DNA of *K. xylinus* as a template and each of primer sets of SEQ ID NOS: 12 and 13, SEQ ID NOS: 14 and 15, and SEQ ID NOS: 16 and 17 as primers, and the amplification product was cloned at an EcoRI restriction enzyme site of a pTSK+ vector by using an In-fusion GD cloning kit (Takara) to prepare a pTSK-(del)2760 vector.

A Ptac::Ec.pfkA gene was amplified by PCR amplification using the pTSa-Ec.pfkA vector as a template and a primer set of SEQ ID NO: 18 and SEQ ID NO: 19 as primers. The PCR product was cloned at an EcoRI restriction enzyme site of a pTSK-(del)2760 vector by using an In-fusion GD cloning kit (Takara) to prepare a pTSK-(del)2760-Ec.pfkA vector.

### 3. Introduction of phosphofructose kinase gene

In order to introduce a nucleotide sequence of SEQ ID NO: 2, which is a pkfA gene of *E.coli,* to *K.xylinus,* a cassette for inserting a Ptac::Ec.pfkA gene was amplified using the pTSK-(del)2760-Ec.pfkA vector as a template and a primer set of SEQ ID NO: 12 and SEQ ID NO: 17 as primers, and the amplification product was introduced to a *K. xylinus* strain by the following transformation. A specific procedure is as follows.

The *K. xylinus* strain was spread on an HS-agar medium (0.5% peptone, 0.5% yeast extract, 0.27% Na₂HPO₄, 0.15% citric acid, 2% glucose, and 1.5% bacto-agar) supplemented with 2% of glucose, and then cultured at 30°C 3 days. The strain was inoculated in a 5 ml HS medium supplemented with 0.2%(v/v) of cellulase (sigma, Cellulase from Trichoderma reesei ATCC 26921), and then cultured at 30°C 2 days. A cell suspension thus cultured was inoculated in a 100 ml HS medium supplemented with 0.2%(v/v) of cellulose so that a cell density (OD₆₀₀) was 0.04, and then the resultant was cultured at 30°C so that a cell density was 0.4 to 0.7. The cultured strain was washed with 1 mM of HEPES buffer, washed three times with 15% of glycerol, and re-suspended with 1 ml of 15% of glycerol to prepare a competent cell.

100 µl of the competent cell thus prepared was transferred to 2 mm of an electro-cuvette, 3 µg of the Ptac::Ec.pfkA cassette prepared in the clause 2 was added thereto, and a vector was introduced to the competent cell by electroporation (2.4 kV, 200 Ω, 25 µF). The vector-introduced cell was re-suspended in 1 ml of a HS medium containing 2% of glucose and 0.1%(v/v) cellulose, transferred to a 14 ml round-bottom tube, and cultured at 30°C and 160 rpm for 16 hours. The cultured cell was spread on a HS medium supplemented with 2% glucose, 1% ethanol, and 5 µg/ml of tetracycline and cultured at 30°C for 4 days. Strains having a tetracycline resistance were selected to prepare PFK gene-over-expressing strains.

### 4. Glucose Consumption and Cellulose and Gluconate Productions

The designated *K.xylinus* strains were inoculated into 50 ml of a HS medium supplemented with 5% glucose and 1% of ethanol, and the resultant was stirred and cultured at 30°C at 230 rpm for 5 days. Then, glucose consumption and the product cellulose were quantified. Glucose and gluconate were analyzed by using HPLC equipped with the Aminex HPX-87H column (Bio-Rad, USA). The product of cellulose was quantified by measuring a weight after washing the cellulose solid produced in the flask with 0.1 N sodium hydroxide solution and distilled water, and freeze-drying the resultant. A gluconate yield was analyzed.

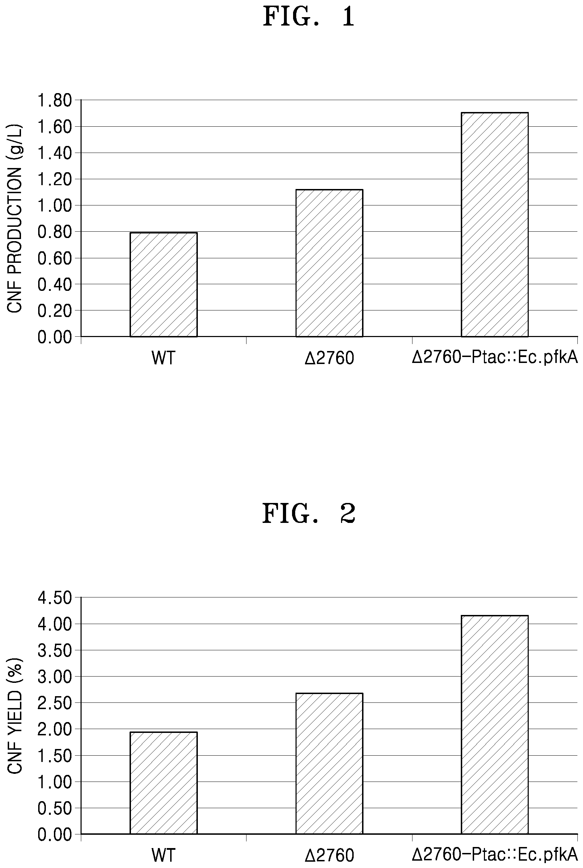

The results are shown in FIGS. 1 to 3. FIG. 1 shows a CNF product obtained from a culture cultured from a *K.xylinus* strain introduced with a PFK gene. As shown in FIG. 1, when the PFKA gene was introduced to *K.xylinus,* the CNF production increased about 115% with respect to a wild-type strain. Table 1 illustrates the data shown in FIGS. 1 and 2.

**[Table 1]**

| | Glucose consumption (g/L) | Gluconate production (g/L) | CNF (g/L) | Gluconate yield (%) | CNF yield (%) |
|---|---|---|---|---|---|
| WT | 40.17 | 29.11 | 0.79 | 72.46 | 1.96 |
| Δ2760 | 41.65 | 31.71 | 1.12 | 76.13 | 2.68 |
| Δ2760-Ptac::Ec.pfkA | 40.76 | 29.85 | 1.70 | 73.24 | 4.18 |

FIG. 2 shows a yield of cellulose nanofibers (CNFs) obtained from the culture prepared by culturing the each of the *K.xylinus* strains introduced with the PFK gene. As shown in FIG. 2, when the PFKA gene was introduced to *K.xylinus,* the CNF yield increased about 113% with respect to a wild-type strain.

Also, the wild-type and recombinant strains were spread on a HSD medium (5 g/L yeast extract, 5 g/L bacto peptone, 2.7 g/L Na₂HPO₄, 1.15 g/L citric acid, and 20 g/L glucose) and a plate containing 20 g/L agar, and the resultant was cultured at 30°C for 3 days.

Starter fermentation was performed by adding 100 mL of a HSD medium in a 250 mL flask, inoculating 3 loops of microorganism, and culturing the resultant at 30°C at 150 rpm for 20 hours.

Main fermentation was performed by using a 1.5 L bench-type fermentor (GX2-series, Biotron) system, a baffle was removed, and a stirring environment with enhanced vertical movement was formed by using a pitch-type impeller and a microsparger.

Operation conditions included an initial volume of 0.7 L, a temperature of 30°C, pH 5.0 (adjusted by using a neutralizing agent 3 N KOH (aq)), a stirring rate of 150 rpm, an airflow amount of 0.7 L/min, a medium, which was a HS medium supplemented with 40 g/L glucose, and inoculation at a rate of 14%(v/v).

In the CMC-added environment, fermentation evaluation included adding Na_CMC 1.0%(w/v) to the same HS medium and changing the stirring rate to 250 rpm from the conditions described above. A CNF quantity was measured based on a weight after pre-treating the collected fermentation solution, that is washing the collected fermentation solution with a 0.1 N NaOH (aq) solution at 90°C for 2 hours.

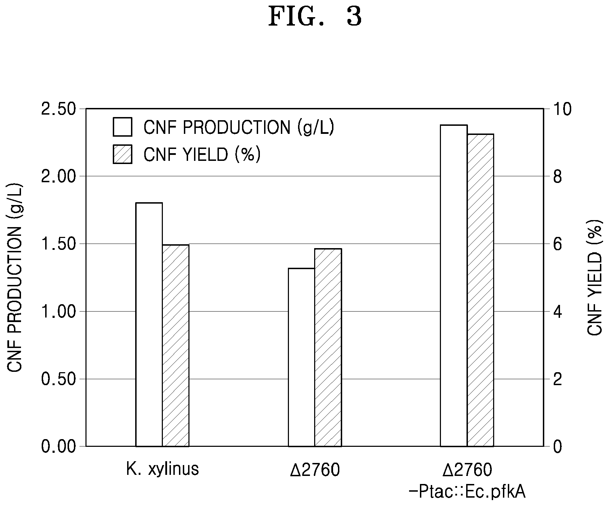

FIG. 3 shows CNF production and yield when a *K.xylinus* strain into which a pfkA gene is introduced was cultured by fermentation. As shown in FIG. 3, when the pfkA gene was introduced to *K.xylinus,* the CNF productions increased about 32%, and the CNF yields increased about 55% than those of the control group. The yield is a percent ratio of the CNF weight produced with respect to a weight of glucose used in the fermentation.

**[Table 2]**

| CMC free fermentation | Glucose consumption (g/L) | CNF production (g/L) | CNF yield (%) |
|---|---|---|---|
| WT | 29.70 | 1.80 | 5.95 |
| Δ2760 | 22.50 | 1.32 | 5.85 |
| Δ2760-Ptac::Ec.pfkA | 25.20 | 2.38 | 9.25 |

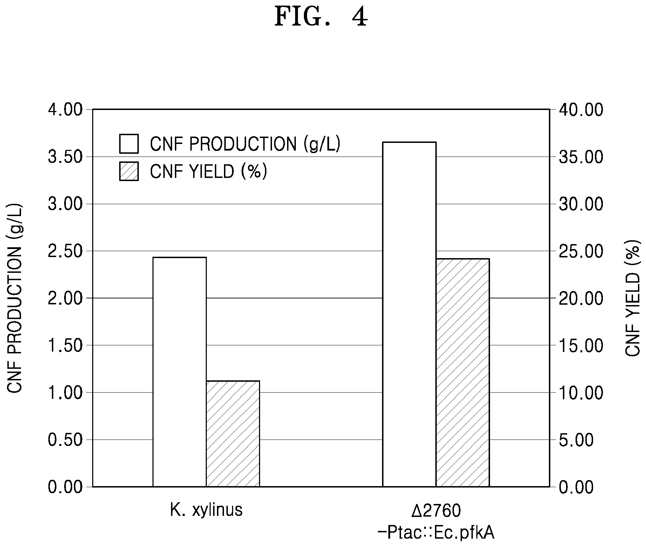

FIG. 4 shows CNF production and yield when a *K.xylinus* strain into which a pfkA gene is introduced was supplemented with CMC and fermented. As shown in FIG. 4, when the pfkA gene was introduced to *K.xylinus,* the CNF productions increased about 50%, and the CNF yields increased about 116% than those of the control group. Table 3 illustrates the data shown in FIG. 4.

**[Table 3]**

| CMC added fermentation | Glucose consumption (g/L) | CNF production (g/L) | CNF yield (%) |
|---|---|---|---|
| WT | 21.7 | 2.43 | 11.18 |
| Δ2760-Ptac:: Ec. pfkA | 15.1 | 3.65 | 24.15 |

This indicates that the introduced exogenous pfkA phosphorylated fructose-6-phosphate of the strain into fructose-1,6-bisphosphate and thus influenced the corresponding reaction and cellulose production.
## Claims

1. A microorganism of the genus *Komagataeibacter* having enhanced cellulose productivity, wherein the microorganism comprises a genetic modification that increases a phosphofructose kinase (PFK) activity, wherein the genetic modification is an increase of the copy number of a gene encoding the PFK or a modification of an expression regulatory sequence of a gene encoding the PFK; preferably wherein the increase of the copy number is caused by introduction of the gene into a cell from an outside of the cell.

2. The microorganism of claim 1, wherein the PFK belongs to the enzyme classified as EC 2.7.1.11.

3. The microorganism of claims 1 or 2, wherein the PFK is a polypeptide having a sequence identity of 90% or higher with an amino acid sequence of SEQ ID NO: 1.

4. The microorganism of any one of claims 1 to 3, wherein the PFK is derived from the genus *Escherichia, Bacillus, Mycobacterium, Zymomonas,* or *Vibrio.*

5. The microorganism of any one of claims 1 to 4, wherein the genetic modification is an increase of the copy number of a gene encoding the PFK or a modification of an expression regulatory sequence of a gene encoding the PFK, and wherein the gene has a nucleotide sequence of SEQ ID NO: 2.

6. The microorganism of any one of claims 1 to 5, wherein the *Komagataeibacter* is *Komagataeibacter xylinus.*

7. A method of producing cellulose, the method comprising:
culturing a recombinant microorganism of the genus *Komagataeibacter* having enhanced cellulose productivity and comprising a genetic modification that increases a PFK activity in a medium to produce cellulose, wherein the genetic modification is an increase of the copy number of a gene encoding the PFK or a modification of an expression regulatory sequence of a gene encoding the PFK; preferably wherein the increase of the copy number is caused by introduction of the gene into a cell from an outside of the cell; and
collecting the cellulose from the culture.

8. The method of claim 7, wherein the PFK is derived from the genus *Escherichia, Bacillus, Mycobacterium, Zymomonas,* or *Vibrio.*

9. The method of claims 7 or 8, wherein the PFK is a polypeptide having a sequence identity of 90% or higher with an amino acid sequence of SEQ ID NO: 1.

10. The method of any one of claims 7 to 9, wherein the genetic modification increases the copy number of a gene encoding a polypeptide that has a sequence identity of 90% or higher with an amino acid sequence of SEQ ID NO: 1 or modifies an expression regulatory sequence of a gene encoding a polypeptide that has a sequence identity of 90% or higher with an amino acid sequence of SEQ ID NO: 1.

11. The method of any one of claims 7 to 10, wherein the *Komagataeibacter* is *Komagataeibacter xylinus.*

12. The method of any one of claims 7 to 11, wherein the medium comprises 0.5%(w/v) to 5.0%(w/v) of carboxymethylcellulose (CMC), 0.1%(v/v) to 5.0%(v/v) of ethanol, or 0.5%(w/v) to 5.0%(w/v) of CMC and 0.1%(v/v) to 5.0%(v/v) of ethanol.

13. A method of producing a microorganism having enhanced cellulose productivity, the method comprising introducing a gene encoding a PFK into a microorganism of the genus *Komagataeibacter.*

## Patentansprüche

1. Ein Mikroorganismus der Gattung *Komagataeibacter* mit verbesserter Zelluloseproduktivität, wobei der Mikroorganismus eine genetische Modifikation umfasst, die die Aktivität einer Phosphofructosekinase (PFK) erhöht, wobei die genetische Modifikation eine Erhöhung der Kopien-Zahl eines Gens ist, das die PFK kodiert, oder eine Modifikation einer Expressions-regulierenden Sequenz eines Gens ist, das die PFK kodiert; wobei die Erhöhung der Kopien-Zahl vorzugsweise durch Einführen eines Gens in eine Zelle von außerhalb der Zelle verursacht wird.

2. Der Mikroorganismus nach Anspruch 1, wobei die PFK zu dem Enzym gehört, das als EC 2.7.1.11 klassifiziert ist.

3. Der Mikroorganismus nach Anspruch 1 oder 2, wobei die PFK ein Polypeptid ist, das eine Sequenzidentität von 90% oder höher mit der Aminosäuresequenz der SEQ ID NR: 1 aufweist.

4. Der Mikroorganismus nach einem der Ansprüche 1 bis 3, wobei die PFK von der Gattung *Escherichia, Bacillus, Mycobacterium, Zymomonas* oder *Vibrio* abgeleitet ist.

5. Der Mikroorganismus nach einem der Ansprüche 1 bis 4, wobei die genetische Modifikation die Erhöhung der Kopien-Zahl eines Gens ist, das die PFK kodiert, oder eine Modifikation einer Expressions-regulierenden Sequenz eines Gens ist, das die PFK kodiert, und wobei das Gen die Nukleotidsequenz der SEQ ID NO: 2 hat.

6. Der Mikroorganismus nach einem der Ansprüche 1 bis 5, wobei das *Komagataeibacter Komagataeibacter xylinus* ist.

7. Ein Verfahren zur Zelluloseproduktion, wobei das Verfahren umfasst:
Züchten eines rekombinanten Mikroorganismus der Gattung *Komagataeibacter* mit verbesserter Zelluloseproduktivität, der eine genetische Modifikation umfasst, die die Aktivität einer PFK erhöht, in einem Medium zur Zelluloseproduktion, wobei die genetische Modifikation eine Erhöhung der Kopien-Zahl eines Gens ist, das die PFK kodiert, oder eine Modifikation einer Expressions-regulierenden Sequenz eines Gens ist, das die PFK kodiert; wobei die Erhöhung der Kopien-Zahl vorzugsweise durch Einführen eines Gens in eine Zelle von außerhalb der Zelle verursacht wird; und
Gewinnen der Zellulose aus der Kultur.

8. Das Verfahren nach Anspruch 7, wobei die PFK von der Gattung *Escherichia, Bacillus, Mycobacterium, Zymomonas* oder *Vibrio* abgeleitet ist.

9. Das Verfahren nach Anspruch 7 oder 8, wobei die PFK ein Polypeptid ist, das eine Sequenzidentität von 90% oder höher mit der Aminosäuresequenz der SEQ ID NR: 1 aufweist.

10. Das Verfahren nach einem der Ansprüche 7 bis 9, wobei die genetische Modifikation die Kopien-Zahl eines Gens erhöht, das ein Polypeptid mit einer Sequenzidentität von 90% oder höher mit der Aminosäuresequenz der SEQ ID NO: 1 kodiert, oder eine Expressions-regulierenden Sequenz eines Gens modifiziert, das ein Polypeptid mit einer Sequenzidentität von 90% oder höher mit der Aminosäuresequenz der SEQ ID NO: 1 kodiert.

11. Das Verfahren nach einem der Ansprüche 7 bis 10, wobei das *Komagataeibacter Komagataeibacter xylinus* ist.

12. Das Verfahren nach einem der Ansprüche 7 bis 11, wobei das Medium 0,5%(w/v) bis 5,0%(w/v) Carboxymethylcellulose (CMC), 0,1%(v/v) bis 5,0%(v/v) Ethanol, oder 0,5%(w/v) bis 5,0%(w/v) CMC und 0,1%(v/v) bis 5,0%(v/v) Ethanol umfasst.

13. Ein Verfahren zum Erzeugen eines Mikroorganismus mit verbesserter Zelluloseproduktivität, wobei das Verfahren das Einführen eines Gens, das eine PFK kodiert, in einen Mikroorganismus der Gattung *Komagataeibacter* umfasst.

## Revendications

1. Micro-organisme du genre *Komagataeibacter* ayant une productivité de cellulose accrue, le micro-organisme comprenant une modification génétique qui augmente une activité de phosphofructose kinase (PFK), la modification génétique étant une augmentation du nombre de copies d'un gène codant pour la PFK ou une modification d'une séquence régulatrice d'expression d'un gène codant pour la PFK ; l'augmentation du nombre de copies étant de préférence causée par l'introduction du gène dans une cellule depuis l'extérieur de la cellule.

2. Micro-organisme selon la revendication 1, la PFK relevant de l'enzyme enregistrée en tant que EC 2.7.1.11.

3. Micro-organisme selon les revendications 1 ou 2, la PFK étant un polypeptide ayant une identité de séquence supérieure ou égale à 90 % vis-à-vis d'une séquence d'acides aminés selon SEQ ID NO : 1.

4. Micro-organisme selon l'une quelconque des revendications 1 à 3, la PFK étant dérivée du genre *Escherichia, Bacillus, Mycobacterium, Zymomonas,* ou *Vibrio.*

5. Micro-organisme selon l'une quelconque des revendications 1 à 4, dans lequel la modification génétique est une augmentation du nombre de copies d'un gène codant pour la PFK ou une modification d'une séquence régulatrice d'expression d'un gène codant pour la PFK, le gène ayant une séquence de nucléotides selon SEQ ID NO : 2.

6. Micro-organisme selon l'une quelconque des revendications 1 à 5, où *Komagataeibacter* est *Komagataeibacter xylinus.*

7. Procédé de production de cellulose, le procédé comprenant :
la culture d'un micro-organisme recombinant du genre *Komagataeibacter* ayant une productivité de cellulose accrue, le micro-organisme comprenant une modification génétique qui augmente une activité de PFK dans un milieu afin de produire de la cellulose, la modification génétique étant une augmentation du nombre de copies d'un gène codant pour la PFK ou une modification d'une séquence régulatrice d'expression d'un gène codant pour la PFK ; l'augmentation du nombre de copies étant de préférence causée par l'introduction du gène dans une cellule depuis l'extérieur de la cellule ; et
le recueil de la cellulose depuis la culture.

8. Procédé selon la revendication 7, dans lequel la PFK est dérivée du genre *Escherichia, Bacillus, Mycobacterium, Zymomonas* ou *Vibrio.*

9. Procédé selon les revendications 7 ou 8, dans lequel la PFK est un polypeptide ayant une identité de séquence supérieure ou égale à 90 % vis-à-vis d'une séquence d'acides aminés selon SEQ ID NO : 1.

10. Procédé selon l'une quelconque des revendications 7 à 9, dans lequel la modification génétique augmente le nombre de copies d'un gène codant pour un polypeptide ayant une identité de séquence supérieure ou égale à 90 % vis-à-vis d'une séquence d'acides aminés selon SEQ ID NO : 1 ou modifie une séquence régulatrice d'expression d'un gène codant pour un polypeptide ayant une identité de séquence supérieure ou égale à 90 % vis-à-vis d'une séquence d'acides aminés selon SEQ ID NO : 1.

11. Procédé selon l'une quelconque des revendications 7 à 10, dans lequel *Komagataeibacter* est *Komagataeibacter xylinus.*

12. Procédé selon l'une quelconque des revendications 7 à 11, dans lequel le milieu comprend de 0,5 % (w/v) à 5,0 % (w/v) de carboxyméthylcellulose (CMC), de 0,1 % (v/v) à 5,0 % (v/v) d'éthanol, ou de 0,5 % (w/v) à 5,0 % (w/v) de CMC et de 0,1 % (v/v) à 5,0 % (v/v) d'éthanol.

13. Procédé de production d'un micro-organisme ayant une productivité de cellulose accrue, le procédé comprenant l'introduction d'un gène codant pour une PFK dans un micro-organisme du genre *Komagataeibacter.*
